# EUROPEAN PATENT SPECIFICATION

(11) **EP 1 910 834 B1**
(45) Date of publication and mention of the grant of the patent: **07.01.2009**
(21) Application number: 06789208.3
(22) Date of filing: 02.08.2006
(51) Int. Cl.: G01N 33/543

(54) **APPARATUS ASSEMBLY AND METHOD FOR DETECTING AN ANALYTE**
GERÄTEANORDNUNG UND VERFAHREN ZUM NACHWEIS EINES ANALYTEN
DISPOSITIF D'APPAREILLAGE ET PROCÉDÉ DE DÉTECTION D'UNE SUBSTANCE À ANALYSER

(30) Priority: 02.08.2005 US 705118 P
(43) Date of publication of application: 16.04.2008
(73) Proprietor: 3M Innovative Properties Company, St. Paul MN 55133-3427 (US)
(72) Inventor: BOMMARITO, G. Marco, 3M Center, Saint Paul, Minnesota 55133-3427 (US); BURTON, Scott A., 3M Center, Saint Paul, Minnesota 55133-3427 (US); DODGE, Larry H., 3M Center, Saint Paul, Minnesota 55133-3427 (US); GONZALEZ, Bernard A., 3M Center, Saint Paul, Minnesota 55133-3427 (US); LAKSHMI, Brinda B., 3M Center, Saint Paul, Minnesota 55133-3427 (US); SMITH, Jeffrey D., 3M Center, Saint Paul, Minnesota 55133-3427 (US)
(74) Representative: Aleandri-Hachgenei, Lorraine E.
(86) International application number: PCT/US2006/030113
(87) International publication number: WO 2007/016633

(56) References cited:
- EP-A1- 0 439 917
- EP-A2- 0 286 371
- US-A- 5 607 863

## Description

### BACKGROUND

Many industries, such as the medical and food service industries, often require the testing of a sample of material in order to determine whether a certain biological bacterium or other organism is present. The presence of such an organism may be indicative of a problem. For example, the presence of the organism may indicate the presence of infection in a person or the presence of a contaminant in food or on a food preparation surface.

In existing methods of testing the sample of material, a sample collection device, such as a swab, which includes a porous medium on the end of a shaft, may be used to gather the sample of material. Specifically, the porous medium of the swab may be placed in contact with a sample source, such as a nose, ear, or throat of a person, or a food preparation surface, and a sample may then adhere to the porous medium. Thereafter, the sample collection device may be transferred to a different location, such as a laboratory, where the collected sample is transferred from the sample collection device to a slide or other external laboratory apparatus in order to run an assay to analyze whether the particular organism of interest is present. The particular organism of interest may be referred to as an "analyte".

In addition to a delay in time, the transfer of the sample collection device from the sample source to the off-site location may cause the collected sample to become contaminated or dry out, which may decrease the reliability of the analyte detection. Furthermore, a non-self contained testing device or method may be problematic because the lab technician may be exposed to the analyte during the testing process. The present invention addresses these and/or other problems and provides advantages over prior devices. EP0286371 A1 discloses a device for lateral-flow immunoassay consisting in a housing which comprises: a fluid reservoir including a liquid with reagents necessary for detecting the analyte, disposed at one end; an aperture for introducing the sample, disposed approximately in the middle; and a test strip with specific antibodies to capture the analyte, disposed between the fluid reservoir and the opposite end of the housing, and below the aperture.

### BRIEF SUMMARY

The application discloses, in one aspect, an apparatus for processing a sample of material. In illustrated embodiments, the apparatus includes a housing having a flow passage between first and second ends. A capture medium is disposed in the housing between the first and second ends and is configured to capture an analyte in a sample of material. In illustrated embodiments, the apparatus is used in combination with a sample collection assembly and an indicator cap having a testing device.

In one aspect, an assembly is disclosed for processing a sample of biological material. The assembly comprises a housing including a first end, a second end opposite the first end and a flow passage between the first and second ends and the first end being configured to receive a sample collection assembly including a fluid reservoir, a second fluid reservoir including a second fluid proximate to the second end of the housing in fluid communication with the flow passage, and a capture medium disposed in the housing between the first end and the second end configured to capture an analyte in a sample of material.

In another aspect, a method is disclosed of processing a sample of biological material. The method comprises introducing a first fluid into a first end of an apparatus housing and eluting at least a portion of the sample of biological material from a sample collection device to form an eluted sample, capturing material from the eluted sample in a capture medium in the housing, rotating the apparatus housing about one-hundred eighty degrees from a sample preparation orientation to a sample testing orientation, and introducing a second fluid into a second end of the apparatus housing to release the material from the capture medium.

The above summary is not intended to describe each disclosed embodiment or every implementation of the present invention. The figures and the detailed description which follow more particularly exemplify illustrative embodiments.

### BRIEF DESCRIPTION OF THE DRAWING

The present invention will be further explained with reference to the drawing figures listed below, where like structure is referenced by like numerals throughout the several views.
FIG. 1 is a perspective view of an exemplary embodiment of an apparatus assembly of the present invention, which includes a sample collection device attached to a first end of a housing, which is in a sample preparation orientation, and an indicator cap, which is configured to attach to the first end of the housing.
FIG. 2 is a perspective view of the apparatus assembly of FIG. 1, where the sample collection device has been detached from the first end of the housing and the indicator cap has been attached to the first end of housing, which is in a testing orientation.
FIG. 3 is a cross-sectional view of the housing of FIG. 1, which is in the sample preparation orientation, where the sample collection device is attached to first end of the housing.
FIG. 4 is a cross-sectional view of the apparatus assembly of FIG. 2, where the housing is in the testing orientation and where the indicator cap is now attached to the first end of the housing.

While the above-identified figures set forth an exemplary embodiment of the present invention, other embodiments are also within the invention. In all cases, this disclosure presents the invention by way of representation and not limitation. It should be understood that numerous other modifications and embodiments can be devised by those skilled in the art, which fall within the scope and spirit of the principles of the invention.

### DETAILED DESCRIPTION

The present invention is an apparatus assembly for detecting an analyte, such as staphylococcus aureus, in a sample of material, where the assembly includes a substantially self-contained housing and an indicator cap. The housing is substantially self-contained because generally all the chemistry for detecting the analyte is contained in the housing. This decreases the chance that an apparatus operator will be exposed to the analyte and/or fluids that are used in the testing process, such as by an accidental spill or otherwise. The inventive apparatus assembly is a relatively simple device that allows a sample of material to be tested for an analyte at or near the sample source. Rather than transferring the sample of material to an off-site laboratory, the present invention allows an operator to obtain a sample of material from a sample source and then in a short amount of time; test the sample for the presence of an analyte at or near the sample source. Furthermore, the apparatus assembly may be disposable, which helps to provide a clean, if not sterile, apparatus assembly for each use.

The housing of the apparatus assembly is configured to receive a sample collection device, such as a swab. In some embodiments of the present invention, the sample collection device may be an element of the apparatus assembly, where the sample collection device is distributed with the housing and indicator cap. In other embodiments, the housing is configured to receive a sample collection device that is provided by the apparatus assembly operator.

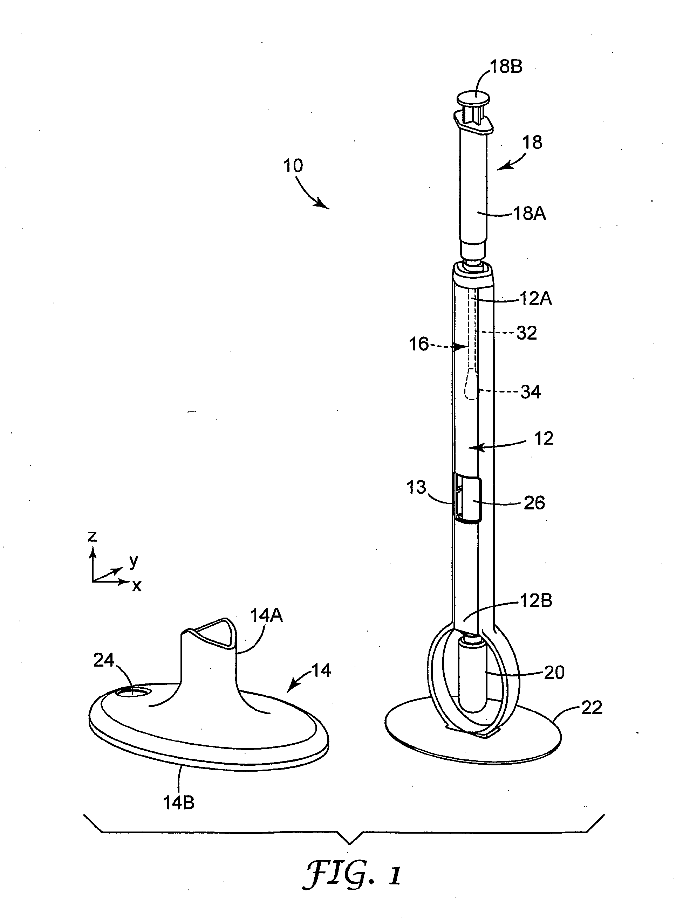

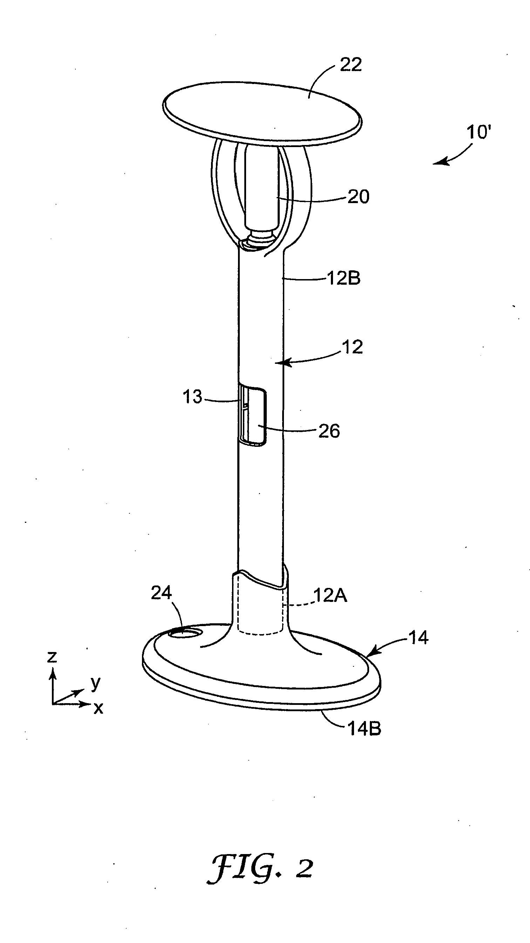

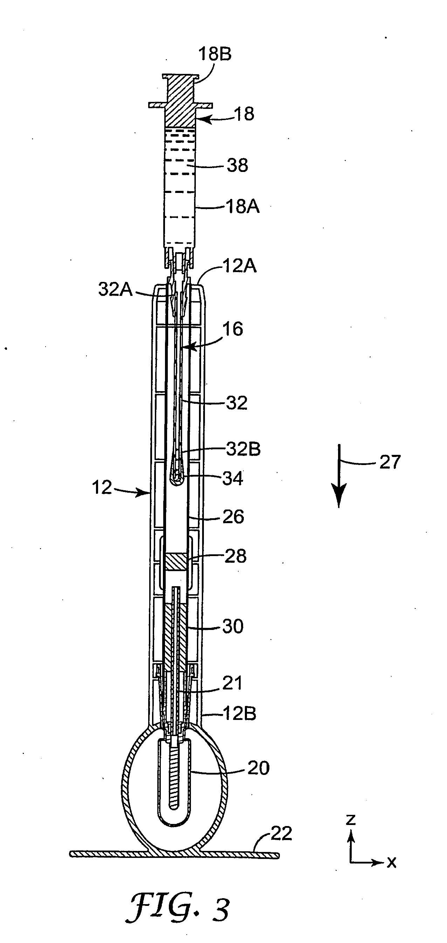

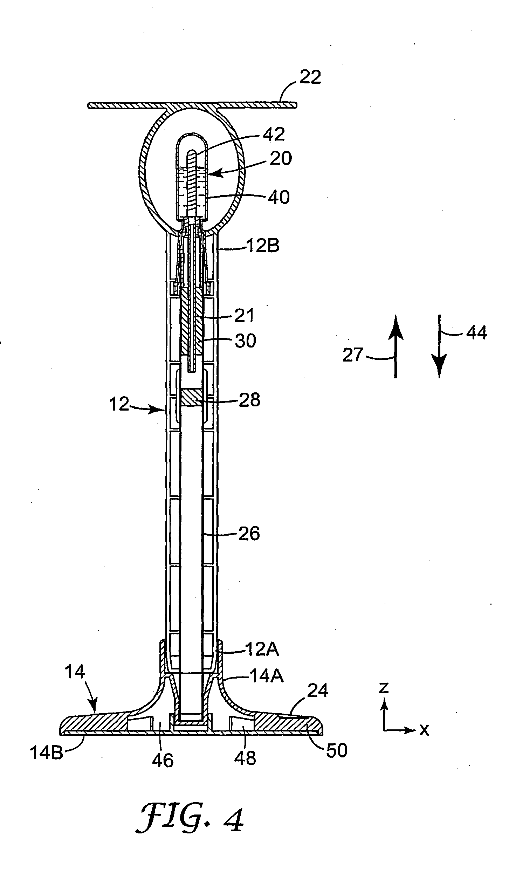

The housing includes two orientations: 1) a sample preparation orientation (shown in FIGS. 1 and 3), and 2) a testing orientation (shown in FIGS. 2 and 4). In the exemplary embodiment, the housing is manually moved between the two orientations by rotating the housing about 180 degrees (°). In the sample preparation orientation, a sample of material is prepared for detection. The sample of material is typically a heterogeneous mixture of material. Certain analytes may need to be isolated from a sample of material and/or concentrated in order for an accurate detection. In the exemplary embodiment, the analyte isolation is completed in the sample preparation orientation. Specifically, a capture medium for isolating the analyte from the sample of material is disposed within the housing in the exemplary embodiment. Preferably, the capture medium is positioned and retained in such a way that fluid may pass over and through the capture medium while at the same time allowing the capture medium to capture the analyte. Examples of suitable capture media include, but are not limited to, beads, a porous membrane, a foam, a frit, a screen, or combinations thereof. The capture medium may be coated with ligand specific to the analyte, e.g., an anti-body. In other embodiments, other means for isolating the analyte may be used.

In the testing orientation, the isolated analyte and a buffer solution contact a testing device, which is adapted to detect presence of the analyte. In the exemplary embodiment, the concentrated analyte is released from the capture medium and contacts a colorimetric sensor, which is adapted to provide a visual indicium of the presence or absence of the analyte.

An exemplary analyte of interest to detect is *Staphylococcus aureus ("S. aureus').* This is a pathogen causing a wide spectrum of infections including: superficial lesions such as small skin abscesses and wound infections; systemic and life threatening conditions such as endocarditis, pneumonia and septicemia; as well as toxinoses such as food poisoning and toxic shock syndrome. Some strains (e.g., Methicillin-Resistant *S*. *aureus* or MRSA) are resistant to all but a few select antibiotics.

The present invention is described in reference to an exemplary embodiment, which uses an indirect assay to detect an analyte in a sample of material. A general understanding of the assay process that is used with the exemplary embodiment will help aid in the description of the inventive apparatus. However, the following description of the assay process is not intended to limit the present invention in any way. Rather, the inventive apparatus and method of detecting an analyte in a sample of material may be applied to many different types of assays, direct or indirect.

In accordance with the exemplary embodiment, a sample of material is obtained with a sample collection device. Prior to running the assay, the sample of material is prepared. In the sample preparation stage, the sample of material is eluted from the sample collection device with a first buffer solution, rendering an eluted sample. At least some of the analyte is then isolated from the eluted sample. This is done with a capture medium. The sample of material is typically a heterogeneous mixture of material. It may be necessary to isolate and, in some sense, concentrate the analyte because some analytes are only detected in large quantities. The isolation/concentration may increase the chance of an accurate detection of the analyte.

Therefore, in order to help increase the possibility that the organism will be detected by a testing device, the organism (i.e., the analyte) is isolated from the remaining debris in the sample of material. The testing device may be any suitable device, such as a colorimetric sensor.

At least some of the analyte captured by the capture medium is then released (or lysed) therefrom with a second buffer solution. The second buffer solution may contain a lysing agent, such as those described in U.S. Patent Application Publication No. 2005/0153370 A1, entitled "Method of Enhancing Signal Detection of Cell-Wall Components of Cells."

The released analyte and second buffer solution is then put in contact with a reagent that is adapted to react with the released analyte. If a direct assay is used, a reagent may not be necessary. After the analyte and reagent react, and after a sufficient "reaction time", the analyte and reagent, along with the second buffer solution, contact the testing device. In an indirect assay, a testing device (i.e., testing device 50 described below) detects the presence of a reagent adapted to react with the analyte, rather than the analyte itself. Specifically, the reagent and analyte react, and then any remaining reagent (i.e., the reagent that has not reacted with the analyte to form a separate product) reacts with the testing device. Thereafter, the testing device provides a visual indicium of the presence and/or quantity of reagent. It is preferred that the analyte and reagent are given sufficient time to react prior to contacting the testing device.

In one embodiment, the reagent reacts with a surface of the testing device (e.g., a red color), and the testing device changes color as the reagent reacts with the testing device. If a large quantity of reagent reacts with the testing device, the testing device may change color, for example, from red to blue. If a small quantity of reagent reacts with the testing device, the testing device may not change color and remain red. The testing device may also be configured to provide an indicium of the quantity of reagent present (which typically represents the quantity of analyte present in the sample of material). For example, the testing device may change color, where the intensity or hue of the color changes depending upon the amount of reagent present. In alternate embodiments, the testing device measures the amount of reagent in another suitable way.

In an indirect assay, the quantity of reagent present indicates the quantity of analyte present because typically, a large quantity of reagent present after the reaction with the analyte indicates that there was not a large quantity of analyte present in the sample of material. Similarly, a small quantity of reagent present after the reaction with the analyte indicates that there was a large quantity of analyte present in the sample of material.

An apparatus assembly of the present invention may be formed of any suitable material, such as polycarbonate or other suitable polymers. It is preferred that the apparatus assembly is disposable, and so a material may be selected based on not only function, but cost.

FIG. 1 is a perspective view of an exemplary embodiment of apparatus assembly 10 of the present invention. Apparatus assembly 10 includes housing 12 and indicator cap 14. Housing 12 includes a sample preparation orientation and a testing orientation. In FIG. 1, housing 12 is in its sample preparation orientation, where a first end 12A of housing 12 has a greater z-coordinate (see orthogonal x-y-z axis in FIG. 1) than second end 12B. The sample preparation orientation will be discussed in greater detail in reference to FIG. 3. The testing orientation of housing 12 is shown in FIG. 2, and will be discussed in greater detail in reference to FIGS. 2 and 4.

Housing 12 includes first end 12A and second end 12B, which are positioned on opposite sides of housing 12. First end 12A of housing 12 is configured to interchangeably receive indicator cap 14 and a sample collection device 16 that is used to collect a sample of material.

In the embodiment illustrated in FIG. 1, the sample collection device 16 includes hollow shaft 32 and porous medium 34 attached to hollow shaft 32 (or swab). Hollow shaft 32 may be handled to contact porous medium 34 with a sample source, thereby adhering a sample of material to porous medium 34. Hollow shaft 32 includes first end 32A and second end 32B opposite first end 32A. First end 32 includes an opening, which is configured to receive first fluid reservoir 18. First fluid reservoir 18 includes first fluid 38, chamber 18A for retaining first fluid 38, and plunger member 18B. First fluid 38 may be a buffer solution. Porous medium 34 is positioned over second end 32B of hollow shaft 32, which includes at least one opening for allowing first fluid 38 to move through second end 32B of hollow shaft 32, and contact porous medium 34.

Sample collection device 16 may be any suitable device. An Example of suitable sample collection devices is described in U.S. Patent No. 5,266,266, entitled, "SPECIMEN TEST UNIT".

The apparatus of the present invention is used to detect an analyte in the sample of material collected using the sample collection device 16. In FIG. 1, the sample collection device 16 is positioned at first end 12A of housing 12 to introduce a sample for detection or processing. In the embodiment shown, the housing 12 includes a sheath 26 that provides a flow passage for fluid through the apparatus 10. In other embodiments, sheath 26 is absent and outer walls of housing 12 form the inner passageway of housing 12. Housing 12 further includes viewing opening 13, through which sheath 26 is in view. Sealed viewing opening 13 in housing 12 (shown in FIG. 1) may be used by an operator to visually verify that fluid is appropriately flowing through apparatus assembly 10.

First fluid reservoir 18 of the sample collection device 16 is positioned in selective fluidic communication with hollow shaft 32 (shown in FIG. 3) of sample collection device 16, where "selective fluidic communication" indicates that there is a valve, plunger (such as in a syringe) or other operator-activated means of introducing first fluid 38 (shown in FIG. 3) disposed in first fluid reservoir 18 into hollow shaft 32 and housing 12. First fluid 38 elutes the sample of material from porous medium 34 of sample collection device 16.

In FIG. 1, first fluid reservoir 18 is formed by a syringe, where first fluid 38 is positioned in a fluid chamber 18A and plunger member 18B may be pushed in order to release first fluid 38 (shown in FIG. 3) from first fluid reservoir 18. In alternate embodiments, first fluid reservoir is a deformable squeeze bulb with a break-off nib (not shown) to control the fluid flow out of first fluid reservoir. In other embodiments, first fluid reservoir is an accordion pleat bulb, or another type of reservoir that can selectively release fluid with greater pressure than a deformable squeeze bulb. A greater amount of pressure may be desirable in order to elute more of the sample of material from the porous medium 34 of sample collection device 16. Certain analytes may be more sensitive and a greater quantity of analyte may be required in order to be detected by the testing device. In those cases, it is preferred that a syringe or other device that is capable of releasing fluid with more pressure is used to release first fluid 38, which elutes the sample of material from sample collection device 16.

After first fluid 38 (shown in FIG. 3) disposed in first fluid reservoir 18 is released, fluid 38 flows through the hollow shaft 32 of the sample collection device 16, thereby eluting at least some of the sample of material from the porous medium 34 of sample collection device 16. This will be described in further detail in reference to FIG. 3.

As shown in FIG. 1, the apparatus 10 also includes a second fluid reservoir 20 and stand 22 proximate to the second end 12B of housing 12. Second fluid 40 (shown in FIG. 4) is disposed in second fluid reservoir 20, and second fluid 40 is used for releasing the analyte from a capture medium is disposed in second fluid reservoir 20, which is in selective fluidic communication with sheath 26, where "selective fluidic communication" indicates that there is a valve, plunger (such as in a syringe) or other user-activated means of introducing fluid 40 (shown in FIG. 4) disposed in second fluid reservoir 20 into sheath 26. This will be described in further detail in reference to FIG. 4. Stand 22 is configured to position housing 12 in a generally upright (i.e., in the generally z-coordinate direction, where orthogonal x-y-z coordinates are shown in FIG. 1) orientation relative to a generally horizontal (i.e., generally in the x-y plane) surface on which stand 22 is placed. As FIG. 1 shows, at least a part of a surface of stand 22 is generally flat in order to rest on a generally horizontal surface.

As shown in FIG. 1, the apparatus assembly 10 includes a testing device for detecting the analyte. In the illustrated embodiment, the testing device is disposed in indicator cap 14 that is selectively coupleable to the housing 12 to test fluid or analyte in housing 12. Preferably, the testing device provides a visual indicium of a test result through window 24. In the exemplary embodiment, the analyte is detected by an indirect assay, and the testing device is a colorimetric sensor. The colorimetric sensor may include a polydiacetylene material, as described in U.S. Patent Application Publication No. 2004/0132217 A1.

The colorimetric sensor provides a visual indicium of the detection or absence of the analyte. Typically, the colorimetric sensor may or may not change color, depending upon whether the analyte is present in the sample of material. A user may view this color change through window 24. The color change may also be graded (e.g., by intensity or hue) in order to indicate the quantity of analyte present. In other embodiments, the color change cannot be detected with a human eye, and a machine or electronic reader, such as a spectrometer, is used to detect the color change. Of course, other testing devices are contemplated, such as a testing device whose indicium of a test result is characterized by a pH change, or some other change in the characteristic of the medium being analyzed.

In the illustrated embodiment, indicator cap 14 includes a lip 14A, which is configured to couple to the first end 12A of housing 12 (after sample collection device 16 has been removed therefrom) to provide a fluid path from the passageway in the housing 12 to a reservoir or testing device in the indicator cap 14.. Bottom portion 14B of indicator cap 14 is configured to position housing 12 in a generally upright orientation relative to a generally horizontal surface on which indicator cap 14 is placed. In this way, indicator cap 14 and stand 22 share a similar function relative to supporting housing 12 relative to a generally horizontal surface.

FIG. 2 is a perspective view of the exemplary embodiment of apparatus assembly 10', which is apparatus assembly 10 of FIG. 1 where sample collection device 16 (shown in FIG. 1) has been detached from first end 12A of housing 12, the housing 12 has been inverted, and indicator cap 14 has been attached to first end 12A of housing 12 for testing the collected sample. Sample collection device 16 may be discarded after it is detached from housing 12. FIG. 2 shows housing 12 in its testing orientation. In order to move housing 12 from its sample preparation orientation to its testing orientation, housing 12 is rotated about 180°, so that now second end 12B of housing 12 has a greater z-coordinate than first end 12A. Fluid 40 (shown in FIG. 4) in second fluid reservoir 20 may now flow through housing 12 by way of gravity. In its testing orientation, stand 22 of housing 12 is no longer positioned to support housing 12 in a generally upright orientation and stand 22 is no longer positioned to rest on a surface on which assembly 10 is placed. Rather, bottom 14B of indicator cap 14 supports housing 12 in a generally upright orientation relative to a surface on which indicator cap 14 is placed.

FIG. 3 is a schematic cross-sectional view of housing 12 of FIG. 1 in a sample preparation orientation, where sample collection device 16 is attached to first end 12A of housing 12. The apparatus 10 includes the sheath 26, capture medium 28, and an absorbent medium 30 in housing 12. In the sample preparation orientation, sheath 26 forms first flow direction 27 relative to housing 12 (designated in FIG. 3 as arrow 27), which is in a generally z-coordinate direction. Capture medium 28 is disposed within sheath 26, between first end 12A and second end 12B of housing 12. Capture medium 28 may be any suitable medium adapted to capture analyte from an eluted sample of material, where the eluted sample is a sample of material that is released from sample collection device 16. Examples of suitable capture media include, but are not limited to, beads coated with an antibody specific to the analyte, a porous membrane, a foam, a frit, a screen, or combinations thereof. In other embodiments, other means for isolating the analyte may be used.

During the sample preparation stage of testing a sample of material, when housing 12 is in a sample preparation orientation, an analyte contained in the sample of material is concentrated. Specifically, at least some of the analyte is isolated from the sample of material. The process of isolating the analyte is best described in reference to a description of the components of housing 12.

After plunger member 18B is moved toward housing 12, first fluid 38 is released from chamber 18A in first fluid reservoir 18, first fluid 38 moves from first end 32A of hollow shaft 32 to second end 32B (in first flow direction 27). As first fluid 38 moves through second end 32B of hollow shaft 32, first fluid 38 contacts porous medium 34 and elutes at least some of the sample of material from porous medium 34, thereby rendering an "eluted sample".

After first fluid 38 elutes at least some of the sample of material porous medium 34, first fluid 38 and the eluted sample move through sheath 26 in first flow direction 27 and contact capture medium 28. The generally vertical (i.e., z-coordinate direction) of sheath 26 allows first fluid 38 to flow through sheath 26 by gravity. In this first flow direction 27, sheath 26 forms a first path. Capture medium 28 captures at least some of the analyte from the eluted sample. Thereafter, first fluid 38 and the eluted sample (minus the captured analyte) then move through sheath 26 and contact absorbing medium 30. Absorbing medium 30 is formed of an absorbent material, which absorbs substantially all of first fluid 38 and the remainder of the eluted sample so that when housing 12 is moved (or rotated) from its sample preparation orientation to the testing orientation, the first fluid and remainder of the eluted sample do not move through sheath 26.

Absorbent medium 30 is disposed in sheath 26 between capture medium 28 and second end 12B of housing 12. Absorbent medium 30 absorbs first fluid 38 and the remainder of the eluted sample (i.e., the eluted sample minus the analyte captured by capture medium 28) after first fluid 38 and the eluted sample move through capture medium 28. In this way, absorbent medium 30 is a means for retaining waste fluid, i.e., first fluid 38 and the remainder of the eluted sample ("debris"). Absorbent medium 30 is configured to retain first fluid 38 and debris in sufficient quantity so that when housing 12 is rotated about 180° from the sample preparation orientation to the testing orientation, little or no first fluid 38 and debris will flow back toward capture medium 28. That is, after first fluid 38 and debris are retained in absorbent medium 30, it is preferred that little to no first fluid 38 and debris flow in a second flow direction relative to housing 12 (designated in FIG. 4 as arrow 44).

In alternate embodiments, other means of retaining waste or first fluid 38 and the remainder of the eluted sample are implemented in sheath 26.

After first fluid 38 is released from first fluid reservoir 18 and first fluid 38 and the eluted sample move through sheath 26, sample collection device 16 may be removed from first end 12A of housing 12 and indicator cap 14 may be attached to first end 12A of housing 12. Thereafter, housing 12 may be moved from its sample preparation orientation to its testing orientation, as shown in FIG. 4. Specifically, housing 12 is rotated about 180°, so that second end 12B of housing 12 has a greater z-coordinate than first end 12A. In the sample preparation orientation, it is unlikely that the isolated analyte disposed in housing 12 will contact indicator cap 14, and therefore, the testing stage typically cannot begin until housing 12 is in its testing orientation. Because the apparatus operator may manually move housing 12 between its sample preparation orientation to its testing orientation, the present invention allows the operator to control when the testing stage begins.

FIG. 4 is a cross-sectional view of housing 12 in the testing orientation, where indicator cap 14 is attached to second end 12B of housing 12. During the preceding steps described above, at least some of the analyte has been isolated from the sample of material and captured in capture medium 28. In order to release (or "lyse") the analyte from capture medium 28, second fluid 40 is introduced into sheath 26 from second fluid reservoir 20, which may be a deformable squeeze bulb or some other suitable selectively activatable fluid dispenser. Second fluid reservoir 20 includes an elongated outlet port 21 in order to help prevent second fluid 40 from contacting absorbent medium 30 as second fluid 40 moves through sheath 26.

Second fluid 40 may be a buffer solution, and when the analyte is staphylococcus aureus, second fluid 40 may contain a lysing agent, such as lysostaphin. In the exemplary embodiment, second fluid reservoir 20 includes snap valve 42, which may be manipulated and broken, thereby releasing second fluid 40 from second fluid reservoir 20. In alternate embodiments, any suitable fluid reservoir may be used for second fluid reservoir 20. For example, if a greater force of fluid is desired than that achieved with a squeeze bulb, a syringe may be substituted for the squeeze bulb of second fluid reservoir 20.

After second fluid 40 is introduced into sheath 26, second fluid 40 moves through sheath 26 in a second flow direction 44 (therefore, sheath forms a second flow path when housing 12 is in the testing orientation). The exemplary embodiment uses an indirect assay to detect the analyte. For an indirect assay, a reagent is mixed with the analyte before the analyte contacts the testing device.

A dehydrated reagent adapted to react with the analyte may be disposed in sheath 26 between second fluid reservoir 20 and capture medium 28. Alternatively, the dehydrated reagent may be disposed in second fluid reservoir 20, such as in outlet port 21, where second fluid reservoir 20 is formed so that the dehydrated reagent and second fluid 40 are unlikely to mix until an operator determined time, such as when the snap valve, or other seal is broken. Of course, if the snap valve or other seal is accidentally broken, the dehydrated reagent and second fluid will likely mix prior to the user-determined time. In yet more alternative embodiments, the reagent may be positioned in capture medium 28 or in indicator cap 14.

Second fluid 40 and the reagent move through capture medium 28, thereby releasing at least some of the analyte from capture medium 28. The analyte and reagent then react as they, along with second fluid 40, move through sheath 26 along second flow direction 44 into third fluid reservoir 46 of indicator cap 14. Third fluid reservoir 46 is configured to receive second fluid 40 and the released analyte. Indicator cap 14 further includes fluid path 48 connecting third fluid reservoir 46 to testing device 50. In the exemplary embodiment, fluid path 48 includes one or more microfluidic elements for controlling the flow of fluid from third fluid reservoir 46 to testing device 50. In the exemplary embodiment, testing device 50 is a colorimetric sensor. In alternate embodiments, other testing devices may be used. Testing device 50 may require fluid to flow past it at or below a certain rate in order for the analyte or reagent in the fluid to react with the testing device. In the case of the exemplary embodiment, an indirect assay is used, and so it is the reagent in the fluid that reacts with the testing device. One or more microfluidic elements may help regulate this rate of fluid flow past testing device 50. In order to encourage fluid flow past testing device 50, an absorbent material may be positioned in indicator cap 14, where testing device 50 is positioned between fluid path 48 and the absorbent material. The absorbent material may help the fluid flow past testing device 50 by way of a wicking action.

After the fluid formed by the second fluid 40, the reagent, and analyte flow past testing device 50 and react therewith, a user may observe the test result in window 24. In the exemplary embodiment, the colorimetric sensor is viewable through window 24. Alternatively, window 24 may be positioned on the underside of indicator cap 14 (i.e., the surface of indicator cap 14 that is opposite lip 14A). The test result indicates whether the analyte is present in the sample of material taken with sample collection device 16 (not shown in FIG. 4), and in some embodiments, the test result indicates the quantity of analyte. The quantity of analyte may, for example, be indicated by a color gradient which corresponds to "low level", "medium level", or "high level" indications. In some embodiments, a label may be positioned near indicator cap 14, where the label provides the operator with a color code. The operator may then compare the color that appears in window 14 with the color code to interpret the test result.

In general, the chemistry of the testing device, first fluid, second fluid, and reagent are dependent upon the particular analyte. Those skilled in the art may modify the chemistry of the apparatus of the present invention in order to adapt the apparatus to a specific analyte.

## Claims

1. An assembly for processing a sample of biological material comprising:
a housing including a first end, a second end opposite the first end and a flow passage between the first and second ends and the first end being configured to receive a sample collection assembly including a first reservoir;
a second fluid reservoir including a second fluid proximate to the second end of the housing in fluid communication with the flow passage; and
a capture medium disposed in the housing between the first end and the second end configured to capture an analyte in a sample of material.

2. The assembly of claim 1, wherein the assembly comprises a reagent adapted to react with the analyte and wherein the reagent is disposed in the capture medium or in the housing between the second end and the capture medium.

3. The assembly of any of the preceding claims, and further comprising:
a fluid collection device in the flow passage between the capture medium and the second end of the housing.

4. The assembly of any of the preceding claims in combination with an indicator cap couplable to the housing proximate to the first end and the indicator cap comprising a testing device adapted to detect presence of the analyte and/or a reagent in the sample of material.

5. The assembly of any of the preceding claims, wherein the testing device comprises a colorimetric sensor for providing a visual indicium of a test result.

6. The assembly of any of the preceding claims, wherein the indicator cap further comprises:
a third fluid reservoir for receiving at least some of the second fluid and the analyte; and
a fluid path that connects the third fluid reservoir and the testing device.

7. The assembly of any of the preceding claims, wherein the second end of the housing comprises a stand adapted to position the housing in a generally upright orientation relative to a generally horizontal surface on which the stand is placed.

8. A method of processing a sample of biological material, the method comprising:
introducing a first fluid into a first end of an apparatus housing and eluting at least a portion of the sample of biological material from a sample collection device to form an eluted sample;
capturing material from the eluted sample in a capture medium in the housing; rotating the apparatus housing about 180 degrees from a sample preparation orientation to a sample testing orientation; and
introducing a second fluid into a second end of the apparatus housing to release the material from the capture medium.

9. The method of claim 8 and further comprising the step of:
testing the material from the eluted sample using a testing device.

10. The method of claim 8 or 9 and further comprising the step of
introducing a reagent to react with an analyte in the capture medium.

## Patentansprüche

1. Anordnung zum Bearbeiten einer Probe biologischen Materials, mit:
einem Gehäuse, das ein erstes Ende, ein zweites Ende gegenüber dem ersten Ende und einen Strömungskanal zwischen dem ersten und zweiten Ende aufweist, wobei das erste Ende derart konfiguriert ist, dass es eine Probensammelanordnung aufweisend ein erstes Reservoir aufnimmt;
einem zweiten Fluidreservoir, das ein zweites Fluid nahe dem zweiten Ende des Gehäuses in Strömungsverbindung mit dem Strömungskanal aufweist; und
einem Auffangmedium, das in dem Gehäuse zwischen dem ersten Ende und dem zweiten Ende angeordnet ist und dazu konfiguriert ist, einen Analyten in einer Probe Material aufzufangen.

2. Anordnung nach Anspruch 1, wobei die Anordnung ein zur Reaktion mit dem Analyten geeignetes Reagenz aufweist und wobei das Reagenz in dem Auffangmedium oder im Gehäuse zwischen dem zweiten Ende und dem Auffangmedium angeordnet ist.

3. Anordnung nach einem der vorhergehenden Ansprüche, ferner mit:
einer Fluidsammeleinrichtung in dem Strömungskanal zwischen dem Auffangmedium und dem zweiten Ende des Gehäuses.

4. Anordnung nach einem der vorhergehenden Ansprüche in Kombination mit einer Anzeigerkappe, die man an das Gehäuse nahe dem ersten Ende koppeln kann, und wobei die Anzeigerkappe eine Testeinrichtung aufweist, die dazu ausgelegt ist, das Vorhandensein des Analyten und/oder eines Reagenz in der Probe Material zu detektieren.

5. Anordnung nach einem der vorhergehenden Ansprüche, wobei die Testeinrichtung einen kolorimetrischen Sensor zur Bereitstellung eines optischen Indizes für ein Testresultat aufweist.

6. Anordnung nach einem der vorhergehenden Ansprüche, wobei die Anzeigerkappe ferner Folgendes aufweist:
ein drittes Fluidreservoir zum Aufnehmen mindestens eines Teils des zweiten Fluids und des Analyten; und
einen Fluidweg, der das dritte Fluidreservoir und die Testvorrichtung verbindet.

7. Anordnung nach einem der vorhergehenden Ansprüche, wobei das zweite Ende des Gehäuses einen Ständer aufweist, der dazu ausgebildet ist, das Gehäuse in einer im Allgemeinen stehenden Orientierung relativ zu einer im Allgemeinen horizontalen Fläche, auf die der Ständer gestellt ist, zu positionieren.

8. Verfahren zum Bearbeiten einer Probe biologischen Materials, wobei das Verfahren Folgendes aufweist:
Einführen eines ersten Fluids in ein erstes Ende eines Vorrichtungsgehäuses und Eluieren mindestens eines Anteils der Probe biologischen Materials aus einer Probensammeleinrichtung zur Bildung einer eluierten Probe;
Auffangen von Material von der eluierten Probe in einem Auffangmedium in dem Gehäuse;
Drehen des Vorrichtungsgehäuses um 180 Grad von einer Probenpräparationsorientierung in eine Probentestorientierung; und
Einführen eines zweiten Fluids in ein zweites Ende des Vorrichtungsgehäuses zum Freigeben des Materials aus dem Auffangmedium.

9. Verfahren nach Anspruch 8 und ferner den folgenden Schritt aufweisend:
Testen des Materials von der eluierten Probe mittels einer Testeinrichtung.

10. Verfahren nach Anspruch 8 oder 9 und ferner den folgenden Schritt aufweisend:
Einführen eines Reagenz zur Reaktion mit einem Analyten in dem Auffangmedium.

## Revendications

1. Ensemble pour traiter un échantillon d'une matière biologique, comprenant:
un boîtier présentant une première extrémité, une deuxième extrémité opposée à la première extrémité et un passage d'écoulement entre les première et deuxième extrémités, et la première extrémité étant configurée de manière à recevoir un ensemble de collecte d'échantillons comprenant un premier réservoir;
un deuxième réservoir de fluide contenant un deuxième fluide à proximité de la deuxième extrémité du boîtier en communication fluidique avec le passage d'écoulement; et
un milieu de capture disposé dans le boîtier entre la première extrémité et la deuxième extrémité et configuré pour capturer une substance à analyser dans un échantillon de matière.

2. Ensemble selon la revendication 1, dans lequel l'ensemble comprend un réactif adapté pour réagir avec la substance à analyser, et dans lequel le réactif est disposé dans le milieu de capture ou dans le boîtier entre la deuxième extrémité et le milieu de capture.

3. Ensemble selon l'une quelconque des revendications précédentes, et comprenant en outre:
un dispositif de collecte de fluide dans le passage d'écoulement entre le milieu de capture et la deuxième extrémité du boîtier.

4. Ensemble selon l'une quelconque des revendications précédentes en combinaison avec un capuchon indicateur pouvant être couplé au boîtier à proximité de la première extrémité, et le capuchon indicateur comprenant un dispositif de test adapté pour détecter la présence de la substance à analyser et/ou d'un réactif dans l'échantillon de matière.

5. Ensemble selon l'une quelconque des revendications précédentes, dans lequel le dispositif de test comprend un capteur colorimétrique destiné à donner un indice visuel d'un résultat de test.

6. Ensemble selon l'une quelconque des revendications précédentes, dans lequel le capuchon indicateur comprend en outre:
un troisième réservoir de fluide destiné à recevoir au moins une partie du deuxième fluide et de la substance à analyser; et
un chemin de fluide qui relie le troisième réservoir de fluide et le dispositif de test.

7. Ensemble selon l'une quelconque des revendications précédentes, dans lequel la deuxième extrémité du boîtier comprend un socle adapté pour positionner le boîtier dans une orientation essentiellement dressée par rapport à une surface sensiblement horizontale sur laquelle le socle est placé.

8. Procédé de traitement d'un échantillon d'une matière biologique, le procédé comprenant les étapes suivantes:
introduire un premier fluide dans une première extrémité d'un boîtier d'appareil et éluer au moins une partie de l'échantillon de matière biologique à partir d'un dispositif de collecte d'échantillons pour former un échantillon élué;
capturer une quantité de matière à partir de l'échantillon élué dans un milieu de capture dans le boîtier;
tourner le boîtier d'appareil de 180 degrés pour le faire passer d'une orientation de préparation d'échantillon à une orientation de test d'échantillon; et
introduire un deuxième fluide dans une deuxième extrémité du boîtier d'appareil de manière à libérer la matière du milieu de capture.

9. Procédé selon la revendication 8 et comprenant en outre l'étape consistant à tester la matière à partir de l'échantillon élué en utilisant un dispositif de test.

10. Procédé selon la revendication 8 ou 9 et comprenant en outre l'étape consistant à introduire un réactif pour provoquer une réaction avec une substance à analyser dans le milieu de capture.
